# EUROPÄISCHE PATENTANMELDUNG

(11) **EP 4 467 109 A1**
(43) Veröffentlichungstag der Anmeldung: **27.11.2024**
(21) Anmeldenummer: 23175583.6
(22) Anmeldetag: 26.05.2023
(51) Int. Cl.: A61F 2/50, A61F 2/58

(54) **VERFAHREN UND VORRICHTUNG ZUM HERSTELLEN EINER PROTHESE FÜR EINEN KÖRPERTEIL EINER GLIEDMASSE**

(71) Anmelder: von Essen, Stefan, 14943 Luckenwalde (DE); von Essen, Willi, 14943 Luckenwalde (DE)
(72) Erfinder: von Essen, Stefan, 14943 Luckenwalde (DE); von Essen, Willi, 14943 Luckenwalde (DE)
(74) Vertreter: Gulde & Partner

(57) **Zusammenfassung**

Die Erfindung betrifft ein Verfahren und eine Vorrichtung (100) zum Herstellen einer Prothese (10) für ein Körperteil einer Gliedmaße.

Es ist vorgesehen, dass je ein digitales Abbild eines beeinträchtigten Körperteils (20) einer Gliedmaße und eines zugehörigen unversehrten Körperteils der Gliedmaße erstellt werden. Auf Basis des digitalen Abbildes des beeinträchtigten Körperteils (20) und des gespiegelten digitalen Abbildes des unversehrten Körperteils wird ein digitales Abbild einer Prothese (22) für das beeinträchtigte Körperteil erstellt. Folglich muss die Prothese (10) nicht von Grund auf neu entworfen werden, sondern kann aufgrund der Chiralität von Gliedmaßen an die Form des unversehrten Körperteils angelehnt werden. Anschließend wird mindestens eine Prothese (10) für das beeinträchtigte Körperteil unter Verwendung des digitalen Abbildes der Prothese (22) und eines 3D-Druckers (106) mit einer Druckauflösung von kleiner als 0,1 mm hergestellt.

## Beschreibung

Die Erfindung betrifft ein Verfahren und eine Vorrichtung zum Herstellen einer Prothese für ein Körperteil einer Gliedmaße. Als Gliedmaße wird bei Menschen und Tieren ein durch Muskeln bewegter paariger Körperanhang genannt. Beispiele hierfür sind Finger, Hände, Arme, Beine, Füße und dergleichen.

Prothesen werden eingesetzt, um fehlende oder eingeschränkte Körperteile zu ersetzen oder zu vervollständigen. Bekanntermaßen wird dabei zunächst ein grober Prothesenrohling erstellt, der durch einen Orthopädietechniker an die Form und Gestalt des betroffenen Körperteils sukzessive und individuell angepasst wird. Der Patient begibt sich häufig mehrmals zum Orthopädietechniker, um die individuell angefertigte Prothese abzustimmen. Insbesondere um lebensechte Prothesen herzustellen, müssen feine Oberflächenstrukturen händisch in die individuelle Prothese eingebracht werden Entsprechend ist der Zeitaufwand zur Herstellung einer individuellen Prothese hoch, wodurch lebensechte Prothesen nicht für jedermann erschwinglich sind. Wird die individuell angefertigte Prothese darüber hinaus beschädigt oder geht verloren, so muss der aufwändige Herstellungsprozess erneut von Grund auf durchgeführt werden.

Daher wäre es wünschenswert, die Herstellung von lebensechten Prothesen zu vereinfachen.

Der Erfindung liegt daher nun die Aufgabe zugrunde, eine Vorrichtung und ein Verfahren zum vereinfachten und kostengünstigen Herstellen von individuellen Prothesen bereitzustellen.

Die erfindungsgemäße Aufgabe wird gelöst durch ein Verfahren und eine Vorrichtung zum Herstellen einer Prothese für ein Körperteil einer Gliedmaße gemäß den unabhängigen Patentansprüchen. Bevorzugte Weiterbildungen sind Gegenstand der jeweils rückbezogenen Unteransprüche.

Ein erster Aspekt betrifft ein Verfahren zum Herstellen einer Prothese für ein Körperteil einer Gliedmaße.

In einem Verfahrensschritt wird ein digitales Abbild eines beeinträchtigten Körperteils einer Gliedmaße und ein digitales Abbild eines zugehörigen unversehrten Körperteils der Gliedmaße erstellt. Ein beeinträchtigtes Körperteil ist beispielsweise ein vollständig oder teilweise fehlendes oder beschädigtes Körperteil. Mit anderen Worten entsprechen die digitalen Abbilder einem digitalen Modell oder Kopie der physischen Körperteile. Bevorzugt werden die digitalen Abbilder unter Verwendung eines 3D-Scanners und/oder eines bildgebenden Verfahrens, wie der Computertomographie (CT), der digitalen Volumentomographie (DVT) und dergleichen, als High-Density Meshes erstellt. Die Güte der digitalen Abbilder ist bevorzugt derart, dass feine Oberflächenstrukturen der Körperteile enthalten sind. Feine Oberflächenstrukturen sind zum Beispiel Strukturen, die in der Breite und/oder Tiefe in einer Auflösung unterhalb von 0,1 mm, beispielsweise von 0,09 mm bis 0,01 mm, bevorzugt von 0,05 mm, unterscheidbar sind.

In einem weiteren Verfahrensschritt wird ein digitales Abbild einer Prothese für das beeinträchtigte Körperteil unter Verwendung eines gespiegelten digitalen Abbildes des unversehrten Körperteils und des digitalen Abbildes des beeinträchtigten Körperteils erstellt. Dadurch, dass Körperteile von Gliedmaßen paarig auftreten und in ihrer Spiegelung nahezu identisch sind (Chiralität), wird erfindungsgemäß die gespiegelte Gestalt des unversehrten Körperteils für die Gestaltung der Prothese des beeinträchtigten Körperteils herangezogen. Aufwändige Gestaltungs- und Anpassungsschritte der Prothese aus einem Rohling sind nicht erforderlich. Außerdem wird eine individuelle Prothese erzeugt, die dem eigenen Körper des Patienten nachgeahmt ist, was eine psychologische Stütze für den Patienten darstellen kann, insbesondere bei einer erst kürzlichen (Teil-)Amputation des Körperteils.

Im einfachsten Fall kann das gespiegelte digitale Abbild des unversehrten Körperteils um die Gestalt des digitalen Abbildes des beeinträchtigten Körperteils reduziert werden, sodass der übrigbleibende Teil das Abbild der Prothese für das beeinträchtigte Körperteil darstellt. Bevorzugt erfolgt dies mittels einer 3D-Bearbeitungssoftware, beispielweise eines Slicer-Programms, wie Geomagic^{®} Freeform^{®} oder Blender. Ebenfalls bevorzugt erfolgen weitere ästhetische und/oder funktionale Anpassungsschritte unter Verwendung einer 3D-Bearbeitungssoftware. Beispielsweise wird das digitale Abbild des beeinträchtigten Körperteils leicht herunterskaliert, um einen engen und sicheren Sitz einer (späteren) Prothese zu gewährleisten. Ferner wird bevorzugt der Übergang des digitalen Abbildes der Prothese zu dem digitalen Abbild des beeinträchtigten Körperteils angepasst und/oder eine Wandstärke des digitalen Abbildes der Prothese vereinheitlicht.

In einem weiteren Verfahrensschritt wird mindestens eine Prothese für das beeinträchtigte Körperteil unter Verwendung des digitalen Abbildes der Prothese und eines 3D-Druckers mit einer Druckauflösung von kleiner als 0,1 mm hergestellt. Bevorzugt wird ein 3D-Drucker mit einer Druckauflösung von kleiner als 0,09 mm, kleiner als 0,08 mm, kleiner als 0,07 mm, kleiner als 0,06 mm oder kleiner als 0,05 mm verwendet. Die Druckauflösung bezieht sich bevorzugt in eine Richtung entlang der Höhenrichtung der Schichtdicke des verwendeten Materials. Durch die Begrenzung der Druckauflösung auf einen Bereich kleiner als 0,1 mm, sind die Oberflächen der Prothese deutlich glatter als bei anderen additiven Fertigungsverfahren, wie beispielsweise bei FDM-Druckern (Fused Deposition Modeling - FDM) oder FFF-Druckern (Fused Filament Fabrication - FFF), die beispielsweise eine Druckauflösung von 0,2 mm bis 0,15 mm ermöglichen. Folglich wird eine besonders lebensechte Prothese bereitgestellt, die einen hohen Tragekomfort und ein verbessertes Tragefühl bei dem Nutzer der Prothese erzeugt. Um noch glattere Oberflächen der Prothesen zu ermöglichen, wird besonders bevorzugt ein 3D-Drucker mit einer Druckauflösung von kleiner als 0,04 mm, kleiner als 0,03 mm, kleiner als 0,025 mm oder kleiner als 0,02 mm verwendet.

Gemäß der vorliegenden Erfindung kann eine lebensechte Prothese für ein Körperteil einer Gliedmaße schnell und unkompliziert hergestellt werden. Dadurch, dass ein digitales Abbild der Prothese vorliegt, kann ohne viel Aufwand eine Vielzahl von Prothesen hergestellt oder nachproduziert werden. Dies ist langfristig kostengünstig möglich. Selbst wenn sich das beeinträchtigte Körperteil im Laufe der Zeit verändert, so kann schnell und unkompliziert auf eine Veränderung des Körperteils reagiert werden, indem das digitale Abbild der Prothese angepasst wird, beispielsweise unter Verwendung eines neu erstellten digitalen Abbildes des beeinträchtigten Körperteils. Ferner können die Prothesen farblich angepasst oder variiert werden, um beispielsweise eine Auswahl von verschiedenfarbigen Prothesen anbieten zu können. Somit können insbesondere jahreszeitbedingte Hauttonveränderungen des Patienten berücksichtigt werden.

In bevorzugter Ausgestaltung ist vorgesehen, dass die Prothese für das beeinträchtigte Körperteil direkt aus dem digitalen Abbild der Prothese durch den Kunstharz-3D-Drucker ausgedruckt wird. Der direkte Druck der Prothese stellt eine einfache und schnelle Methode dar, die Prothese gemäß ihrem digitalen Abbild physisch herzustellen.

In weiterer bevorzugter Ausgestaltung ist vorgesehen, dass eine Positiv-Form der Prothese und eine Positiv-Form des beeinträchtigten Körperteils unter Verwendung der digitalen Abbilder der Prothese und des beeinträchtigten Körperteils und unter Verwendung des Kunstharz-3D-Druckers erzeugt wird. Ferner wird ein Negativ-Abdruck der Prothese unter Verwendung der Positiv-Form der Prothese erzeugt und die Prothese für das beeinträchtigte Körperteil unter Verwendung des Negativ-Abdrucks der Prothese hergestellt. Mit anderen Worten wird die Prothese für das beeinträchtigte Körperteil hierbei indirekt aus dem digitalen Abbild der Prothese durch den Kunstharz-3D-Drucker ausgedruckt. Durch das Erstellen eines Negativ-Abdrucks der Prothese können die Farbvielfalt erhöht und die Kosten zur Herstellung der Prothese oder einer Vielzahl von Prothesen gesenkt werden, da die hergestellte Prothese nicht auf die durch die Kunstharze des 3D-Druckers vorgegebenen Farben angewiesen ist. Folglich können im Vergleich zum Kunstharz günstigere Werkstoffe zur Herstellung der Prothese verwendet werden. Bevorzugt wird als Werkstoff Silikonkautschuk verwendet. Silikonkautschuke sind besonders hautverträglich, gut verarbeitbar und günstig. Bevorzugt wird Silikon in den Negativ-Abdruck der Prothese gegossen und die Positiv-Form des beeinträchtigten Körperteils in den Negativ-Abdruck der Prothese eingelegt beziehungsweise eingepresst, um den passgenauen Übergang der Prothese zu dem beeinträchtigten Körperteil in dem Negativ-Abdruck auszuformen. Der Negativ-Abdruck kann mehrfach verwendet werden, sodass auch hier einfach eine Vielzahl von Prothesen hergestellt werden kann. Durch entsprechende Färbungen der Silikone kann analog eine Vielzahl von unterschiedlichen Hauttönen abgedeckt werden. Auf Veränderungen des beeinträchtigten Körperteils kann analog reagiert werden, indem das digitale Abbild des beeinträchtigten Körperteils erneuert und als neue Positiv-Form des beeinträchtigten Körperteils unter Verwendung des 3D-Druckers ausgedruckt wird. Durch das Einpressen der neuen Positiv-Form in das sich in dem Negativ-Abdruck befindlichen Silikon kann die Prothese mit einem angepassten neuen Übergang zum beeinträchtigten Körperteil ausgestaltet werden.

Bevorzugt werden kalt- (Raumtemperatur vernetzend - RTV) und/oder heißvernetzende (Hochtemperatur vernetzend - HTV) Silikonkautschuke und/oder Flüssig-Silikone (*Liquid Silicone Rubber -* LSR) verwendet. Die Shore-Härte der ausgehärteten Silikone beträgt bevorzugt A 30 bis A 80, vorzugsweise A 30 bis A 60, besonders bevorzugt A 50, gemessen nach DIN ISO 48-4. Beispiel für RTV-Silikone sind REPISIL 35 NO A/B mit einer Shore Härte von A 35 (nach DIN ISO 868) und SF 45 - RTV 2 mit einer Shore Härte von A 45 (nach DIN ISO 48-4). Ein Beispiel für ein Flüssig-Silikon ist ELASTOSILO KR 5040/50 A/B mit einer Shore Härte A 50 (nach DIN ISO 48-4).

Bevorzugt wird beim Herstellen der Prothese für das beeinträchtigte Körperteil ein Textilstoff in die hergestellte Prothese eingebettet. Der eingebettete Textilstoff dient der Verstärkung und Erhöhung der Reißfestigkeit des Silikons. Hierdurch kann zudem eine Wandstärke des Silikons reduziert werden, wodurch Materialkosten und Gewicht der Prothese eingespart werden. Bevorzugt wird ein Strumpf aus Textilstoff in den Negativ-Abdruck der Prothese eingespannt und das Silikon in den Negativ-Abdruck der Prothese eingegossen. Durch das Einpressen der Positiv-Form des beeinträchtigten Körperteils wird sichergestellt, dass das Silikon den Strumpf umhüllt. Bevorzugt wird ein Nylonstrumpf verwendet. Nylon ist besonders reißfest.

Ebenfalls bevorzugt werden Luftlöcher und/oder Formschlösser in die digitalen Abbilder des beeinträchtigten Körperteils und der Prothese eingefügt. Die Luftlöcher (auch Luftkanäle) ermöglichen eine bessere Entlüftung des Silikons in dem Negativ-Abdruck der Prothese. Das Anbringen von Formschlössern gewährleistet den korrekten Sitz der Positiv-Form des beeinträchtigten Körperteils in dem Negativ-Abdruck der Prothese, wodurch die Herstellung vereinfacht wird. Bevorzugt sind die Formschlösser derart ausgestaltet, dass nur genau eine Anordnungsmöglichkeit besteht.

In weiterer bevorzugter Ausgestaltung ist vorgesehen, dass die Prothese für das beeinträchtigte Körperteil ein Entlastungskissen aus einem weichen Silikon umfasst. Das Entlastungskissen dient als Puffer zwischen der Prothese und dem Körperteil, wodurch das Tragegefühl und der Tragekomfort des Patienten verbessert wird. Das weiche Silikon umfasst bevorzugt eine Shore-Härte von 00-10 bis A 30, vorzugsweise 00-20 bis A 5, besonders bevorzugt 00-30 bis 00-50 oder 00-20 bis 00-30, gemessen nach DIN ISO 48-4. Beispiele für weiche Silikone sind die additionsvernetzenden Silikone der ECOFLEX^{®} Serie.

Bevorzugt wird das digitale Abbild des beeinträchtigten Körperteils um eine vorgesehene Dicke des Entlastungskissens verlängert. Beim Herstellen der Prothese für das beeinträchtigte Körperteil unter Verwendung des Negativ-Abdrucks der Prothese wird dann ein erstes Silikon in den Negativ-Abdruck eingegossen, die Positiv-Form des verlängerten beeinträchtigten Körperteils in den Negativ-Abdruck eingepresst und nach Aushärtung des ersten Silikons ein zweites Silikon auf das erste Silikon in dem Negativ-Abdruck gegossen. Hierbei ist das zweite Silikon weicher als das erste Silikon. Dies ermöglicht eine einfache Herstellung einer Prothese mit einem einstückigen Entlastungskissen. Die Einstückigkeit ist insofern gegeben, als dass das zweite Silikon bei der Vernetzung an dem ersten ausgehärteten Silikon anhaftet. Das erste Silikon ist bevorzugt das eingangs genannte Silikon, während das zweite Silikon bevorzugt das als weich bezeichnete Silikon ist.

In weiterer bevorzugter Ausgestaltung ist vorgesehen, dass eine Farbtonbestimmung der Gliedmaße erfolgt und die Prothese in einer Farbgebung basierend auf dem bestimmten Farbton hergestellt wird. Bevorzugt wird ein Farbscanner-System zum Ermitteln des Hauttons verwendet. Beispiele hierfür sind tragbare Farbsensoren von Spectromatch oder Nix Pro. Zusätzlich oder alternativ können Referenzfotos der Gliedmaßen verwendet werden. Durch den bestimmten Farbton der Gliedmaße kann eine Prothese hergestellt werden, die dem Hautbild des Patienten möglichst nahe kommt, um eine lebensechte Gestaltung zu erhalten. Hierdurch kann sich die Akzeptanz des Patienten und somit der Tragekomfort der Prothese verbessern. Bevorzugt wird ein äußeres Erscheinungsbild des unversehrteren Körperteils durch Aufbringen von Farbe auf der Prothese imitiert. Dies kann mittels Airbrushpistole, Pinsel oder dergleichen erfolgen.

In weiterer bevorzugter Ausgestaltung ist vorgesehen, dass der verwendete 3D-Drucker ein SLS- oder Kunstharz-3D-Drucker ist. SLS-3D-Drucker (Selective Laser Sintering) zeichnen sich durch eine hohe Auflösung und durch im Wesentlichen nicht (für das menschliche Auge) sichtbare Schichtübergange, d.h. keine einzelnen sichtbaren Schichten, aus. Bei der Verwendung des SLS-3D-Druckers wird eine Schicht feinkörniges Pulver in den Drucker eingegeben und durch einen Laser zu einem Teil verschmolzen. Kunstharz-3D-Drucker zeichnen sich durch ihre besondere Druckqualität aus. Kunstharz-3D-Drucker verwenden 3D-Druckverfahren wie Stereolithografie (SLA) oder digitale Lichtverarbeitung (Digital Light Processing - DLP), wodurch die Auflösung durch den optischen Punkt des Lasers oder des Projektors (SLA) beziehungsweise des Pixels (DLP) bestimmt wird. Da Licht für die Polymerisation verwendet wird, wird beim Drucken keine Kraft angewendet, wodurch die Oberflächen deutlich glatter sind als bei anderen additiven Fertigungsverfahren, wie beispielsweise bei FDM-Druckern (Fused Deposition Modeling - FDM) oder FFF-Druckern (Fused Filament Fabrication - FFF). Insbesondere können mit SLS-3D-Druckern Schichtdicken, also eine Druckauflösung, unterhalb von 0,08 mm, beispielsweise von 0,04 mm bis 0,08 mm, und mit Kunstharz-3D-Druckern Schichtdicken, also eine Druckauflösung, unterhalb von 0,025 mm, beispielsweise zwischen 0,025 mm bis 0,01 mm, erreicht werden. In einer weiteren Ausführungsform wird bevorzugt ein 3D-Drucker auf der Basis von zweifarbigen Photoinitiatoren verwendet. Hierbei wird ein zweifarbige Photoinitiatoren enthaltenes Material aus unterschiedlichen Richtungen mit Licht unterschiedlicher Wellenlänge entsprechend den Absorptionsfarben der Photoinitiatoren beleuchtet und dort wo das Licht der unterschiedlichen Wellenlänge auf einen zweifarbigen Photoinitiator trifft, erfolgt selektiv eine Materialisierung, sodass ein zu erzeugendes 3D-Modell durch aus dem Modell gewonnene Schnittbilder erzeugt werden kann.

In weiterer bevorzugter Ausgestaltung ist vorgesehen, dass eine Größe des digitalen Abbildes der Prothese und/oder des digitalen Abbildes des beeinträchtigten Körperteils derart angepasst wird, dass die hergestellte Prothese mittels Saugeffekt an dem beeinträchtigten Körperteil befestigbar ist. Beispielsweise wird die Größe eines der digitalen Abbilder um 90 Prozent bis 110 Prozent angepasst, vorzugsweise um 92 Prozent bis 108 Prozent. Insbesondere bei kleineren Gliedmaßen, wie Fingern, kann so ein ausreichender Halt sichergestellt werden, sodass auf zusätzliche Befestigungsmittel verzichtet werden kann. Bei größeren Gliedmaßen können dem Fachmann grundsätzlich bekannte Befestigungsvorrichtungen, wie Laschen, Rastelemente und dergleichen, vorgesehen sein.

In weiterer bevorzugter Ausgestaltung ist vorgesehen, dass ein Negativ-Abdruck des beeinträchtigten Körperteils und ein Negativ-Abdruck des zugehörigen unversehrten Körperteils erzeugt wird. Ferner werden eine Positiv-Form des beeinträchtigten Körperteils und eine Positiv-Form des unversehrten Körperteils unter Verwendung der Negativ-Abdrücke erzeugt. Hierbei werden die digitalen Abbilder des beeinträchtigten Körperteils und des unversehrten Körperteils unter Verwendung der Positiv-Formen erstellt. Unter Verwendung von Negativ-Abdrücken können feine Oberflächenstrukturen auf die Positiv-Formen übertragen werden. Hierbei müssen lediglich die Negativ-Abdrücke des Patienten erstellt werden, welche dann eine ortsunabhängige Erzeugung von digitalen Abbildern ermöglichen. Beispielsweise werden die Negativ-Abdrücke des Patienten erstellt und an einen anderen Ort versendet, um dort erfindungsgemäß verwendet zu werden. Folglich muss der Patient nicht zur Erstellung der digitalen Abbilder anwesend sein. Zudem tritt kein durch die Bewegung des Patienten verursachtes Verwackeln bei der Erstellung der digitalen Abbilder auf. Bevorzugt werden die Negativ-Abdrücke mittels Silikon, Alginat, oder Gips hergestellt. Die Positiv-Formen werden bevorzugt mit Gips, Polyurethane (PU), Polymergips oder einem ähnlichen Material unter Verwendung der Negativ-Abdrücke ausgegossen.

In weiterer bevorzugter Ausgestaltung ist vorgesehen, dass die hergestellte Prothese einen Kern aus einem Silikongemisch mit Füllstoffen umfasst. Unter Verwendung eines Kerns mit Füllstoffen können 25 Prozent bis 33 Prozent Gewicht bei der Prothese eingespart werden. Eine Wandstärke der Mantelschicht der Prothese beträgt dann bevorzugt zwischen 0,5 cm und 1 cm. Besonders vorteilhaft können Kerne aus Silikongemischen mit Füllstoffen für Fußprothesen, insbesondere Vorderfußprothesen verwendet werden. Bei einer Vorderfußprothese einer erwachsenen Person kann so beispielsweise ein Gewicht in der Größenordnung von 400 g eingespart werden.

In weiterer bevorzugter Ausgestaltung ist vorgesehen, dass in dem digitalen Abbild der Prothese ein ausgesparter Bereich für ein einzusetzendes Objekt vorgesehen ist. Beispielsweise wird ein Finger- oder Fußnagel in dem digitalen Abbild entfernt, damit die hergestellte Prothese einen entsprechenden Freiraum aufweist, um einen Kunstfingernagel oder -fußnagel in dem Freiraum anzubringen. Bevorzugt werden Acrylnägel verwendet. Durch das Aufbringen von Objekten, wie Fuß- oder Fingernägeln, wird die Prothese noch lebensechter gestaltet, wodurch der Tragekomfort steigt. Zusätzlich oder alternativ können auch ästhetische Makel entfernt werden.

Ein weiterer Aspekt betrifft eine Vorrichtung zum Herstellen einer Prothese für ein Körperteil einer Gliedmaße. Die Vorrichtung umfasst eine Erfassungseinheit, die zum Erstellen von digitalen Abbildern von beeinträchtigten und unversehrten Körperteilen einer Gliedmaße eingerichtet ist. Die Erfassungseinheit ist bevorzugt ein 3D-Scanner, der dazu eingerichtet ist, High-Density Meshes zu erstellen. Die Vorrichtung umfasst ferner eine elektronische Steuereinheit, die dazu eingerichtet ist, ein digitales Abbild einer Prothese für das beeinträchtigte Körperteil unter Verwendung eines gespiegelten digitalen Abbildes des unversehrten Körperteils und des digitalen Abbildes des beeinträchtigten Körperteils zu erstellen, und einen Kunstharz-3D-Drucker, der dazu eingerichtet ist, eine Prothese für das beeinträchtigte Körperteil unter Verwendung des digitalen Abbildes der Prothese herzustellen und/oder Positiv-Formen der digitalen Abbilder der Prothese und des beeinträchtigten Körpertals unter Verwendung der digitalen Abbilder der Prothese und des beeinträchtigten Körperteils. Die mit den Verfahren beschriebenen optionalen Merkmale und deren Vorteile lassen sich analog mit der Vorrichtung verwirklichen und sind daher beliebig miteinander kombinierbar.

In bevorzugter Ausgestaltung ist vorgesehen, dass die Vorrichtung ferner einen Formbaukasten zum Anfertigen von Negativ-Abdrücken umfasst.

Die oben genannte Steuereinheit ist bevorzugt durch elektrische oder elektronische Bauteile oder Komponenten (Hardware) oder durch Firmware (ASIC) implementiert. Zusätzlich oder alternativ wird die Funktionalität der Steuereinheit beim Ausführen eines geeigneten Programms (Software) verwirklicht. Ebenfalls bevorzugt ist die Steuereinheit durch eine Kombination von Hardware, Firmware und/oder Software verwirklicht. Beispielsweise sind einzelne Komponenten der Steuereinheit zum Bereitstellen einzelner Funktionalitäten als separat integrierter Schaltkreis ausgebildet oder auf einem gemeinsamen integrierten Schaltkreis angeordnet.

Die einzelnen Komponenten der Steuereinheit sind ferner bevorzugt als ein oder mehrere Prozesse ausgebildet, die auf einem oder mehreren Prozessoren in einem oder mehreren elektronischen Rechengeräten laufen und beim Ausführen von einem oder mehreren Computerprogrammen erzeugt werden. Die Anweisungen der Computerprogramme sind dabei bevorzugt in einem Speicher abgelegt, wie beispielsweise einem RAM-Element. Die Computerprogramme können jedoch auch in einem nicht-flüchtigen Speichermedium, wie beispielsweise einer CD-ROM, einem Flash-Speicher oder dergleichen, abgelegt sein.

Dem Fachmann ist ferner ersichtlich, dass die Funktionalitäten von mehreren Recheneinheiten (Datenverarbeitungsgeräten) kombiniert oder in einem einzigen Gerät kombiniert sein können oder dass die Funktionalität von einem bestimmten Datenverarbeitungsgerät auf eine Vielzahl von Geräten verteilt vorliegen kann, um die Funktionalität der Steuereinheit zu verwirklichen.

Ein weiterer Aspekt betrifft ein Computerprogramm, umfassend Befehle, die bei der Ausführung des Programms durch einen Computer, wie beispielsweise einer Vorrichtung zum Herstellen einer Prothese für ein Körperteil einer Gliedmaße, diesen veranlassen, das erfindungsgemäße Verfahren, insbesondere ein Verfahren zum Herstellen einer Prothese für ein Körperteil einer Gliedmaße, durchzuführen.

Weitere bevorzugte Ausgestaltungen der Erfindung ergeben sich aus den übrigen, in den Unteransprüchen genannten Merkmalen.

Die verschiedenen in dieser Anmeldung genannten Ausführungsformen der Erfindung sind, sofern im Einzelfall nicht anders ausgeführt, mit Vorteil miteinander kombinierbar.

Die Erfindung wird nachfolgend in Ausführungsbeispielen anhand der zugehörigen Zeichnungen erläutert. Es zeigen:
- Figur 1: eine schematische Darstellung eines Verfahrens gemäß einer Durchführungsform;
- Figur 2: eine schematische Darstellung eines Negativ-Abdrucks eines beeinträchtigten Körperteils und eines Negativ-Abdrucks eines unversehrten Körperteils;
- Figur 3: eine schematische Darstellung einer Positiv-Form eines beeinträchtigten Körperteils und einer Positiv-Form des zugehörigen unversehrten Körperteils;
- Figur 4: eine schematische Darstellung eines digitalen Abbilds des beeinträchtigten Körperteils und eines digitalen Abbilds der Prothese;
- Figur 5: eine schematische Darstellung einer Positiv-Form der Prothese und einer Positiv-Form des beeinträchtigten Körperteils;
- Figur 6: eine schematische Darstellung einer Prothese für ein Körperteil einer Gliedmaße; und
- Figur 7: eine schematische Darstellung einer Vorrichtung gemäß einer Ausführungsform.

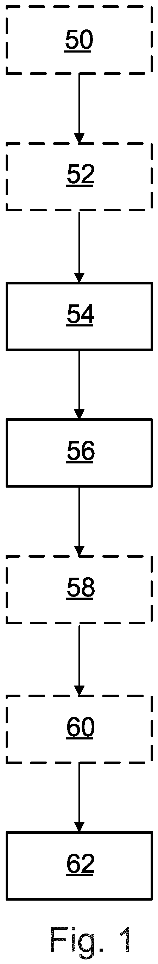

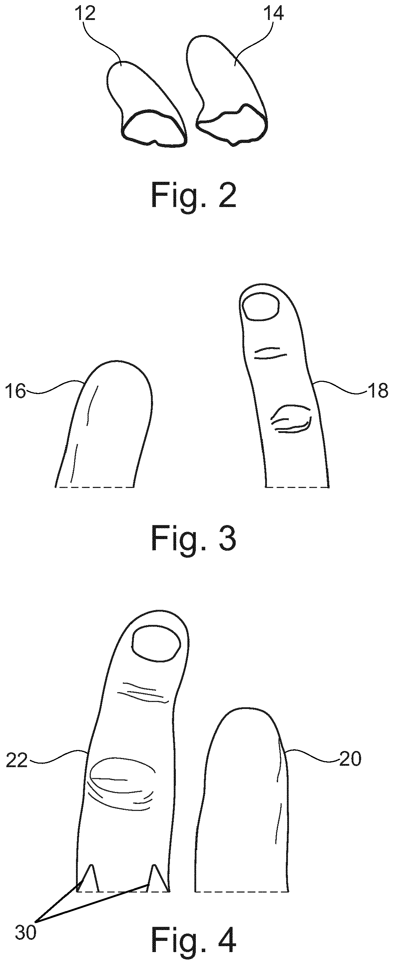

Figur 1 zeigt eine schematische Darstellung eines Verfahrensablaufs zum Herstellen einer Prothese 10 für ein Körperteil einer Gliedmaße gemäß einer Durchführungsform. Das in Figur 1 dargestellte Verfahren wird im Hinblick auf die Figuren 2 bis 6 näher anhand eines Fingerpaares als Körperteile einer Gliedmaße erläutert, ohne darauf beschränkt zu sein. Vielmehr soll lediglich die zu Grunde liegende erfinderische Idee veranschaulicht werden. Das Verfahren erlaubt es, individuelle Prothesen 10 einfach und kostengünstig herzustellen.

In einem ersten Verfahrensschritt 50 wird ein Negativ-Abdruck eines beeinträchtigten Körperteils 12 und ein Negativ-Abdruck des zugehörigen unversehrten Körperteils 14 erzeugt (siehe Figur 2). Hierzu wird Silikon, Alginat oder Gips auf den Fingerstumpf und den zugehörigen gegenüberliegenden unversehrten Finger des Patienten aufgetragen und die Negativ-Abdrücke 12, 14 hergestellt. Die Negativ-Abdrücke 12, 14 übernehmen dabei die feinen Oberflächenstrukturen der beiden Finger des Patienten. Nach Erstellung der Negativ-Abdrücke 12, 14 ist eine weitere Anwesenheit des Patienten nicht mehr erforderlich, sodass die weitere Herstellung der Prothese 10 ohne den Patienten erfolgen kann. Dadurch ist der Aufwand für den Patienten äußerst gering. Beispielsweise können die am Patienten genommenen Negativ-Abdrücke 12, 14 zur weiteren Verarbeitung an ein spezialisierten Labor, beispielsweise länderübergreifend, übersendet werden.

In einem zweiten Verfahrensschritt 52 werden eine Positiv-Form des beeinträchtigten Körperteils 16 und eine Positiv-Form des unversehrten Körperteils 18 unter Verwendung der Negativ-Abdrücke 12, 14 erzeugt (siehe Figur 3). Hierzu wird Gips, Polyurethane (PU) oder Polymergips in die Negativ-Abdrücke 12, 14 gepresst, damit die feinen Oberflächenstrukturen auf die Positiv-Formen 16, 18 übertragen werden. So bleiben die Informationen über den individuellen Fingernagel, die Fingerkuppen- und Knöchelformen und die charakteristischen Haut- und Knickfalten des Fingers des Patienten erhalten. Da die Positiv-Formen 16, 18 als physische Kopie des Fingerpaares des Patienten dienen, sind der Erhalt der charakteristischen Ausgestaltung des Fingers und der feinen Oberflächenstrukturen wichtig, um eine möglichst lebensechte Prothese 10 herstellen zu können.

Anschließend wird ein digitales Abbild eines beeinträchtigten Körperteils 20 einer Gliedmaße (siehe Figur 4) und ein digitales Abbild eines zugehörigen unversehrten Körperteils der Gliedmaße (nicht dargestellt) erstellt (dritter Verfahrensschritt 54). Hierbei werden die digitalen Abbilder 20 unter Verwendung der Positiv-Formen 16, 18 erstellt. Mit einem 3D-Scanner oder einem bildgebenden Verfahren, wie CT oder DVT, werden High-Density Meshes der beiden Finger erstellt. Die digitalen Abbilder 20 sind so detailgetreu erstellt, dass die charakteristische Ausgestaltung und die feinen Oberflächenstrukturen der Fingers enthalten sind. So können Strukturen bis 0,01 mm voneinander unterschieden, also aufgelöst, werden.

In einem vierten Verfahrensschritt 56 wird ein digitales Abbild einer Prothese 22 für das beeinträchtigte Körperteil unter Verwendung eines gespiegelten digitalen Abbildes des unversehrten Körperteils und des digitalen Abbildes des beeinträchtigten Körperteils 20 erstellt (siehe erneut Figur 4). Erfindungsgemäß wird ausgenutzt, dass Finger in der Natur paarig auftreten und die Fingerpaare in ihrer Spiegelung nahezu identisch sind (Chiralität). Somit kann eine Prothese für einen Fingerstumpf, wie in dem vorliegenden Beispiel gewählt, auf Basis der gespiegelten Gestalt des unversehrten Fingers hergestellt werden. Dadurch, dass das digitale Abbild des unversehrten Fingers vorliegt, kann dieser kinderleicht digital gespiegelt werden. Folglich wird einfach und kostengünstig eine individuelle Prothese entworfen, die dem eigenen Körper des Patienten nachgeahmt ist. Die Nachahmung des eigenen Körpers kann eine psychologische Stütze für den Patienten darstellen, vor allem bei einer erst kürzlichen Amputation oder eines kürzlichen Verlustes des Fingers. Dadurch, dass der unversehrte Finger so detailgenau digital abgebildet wird, kann der Effekt durch eine entsprechend detailgenau hergestellte Prothese 10 verstärkt werden.

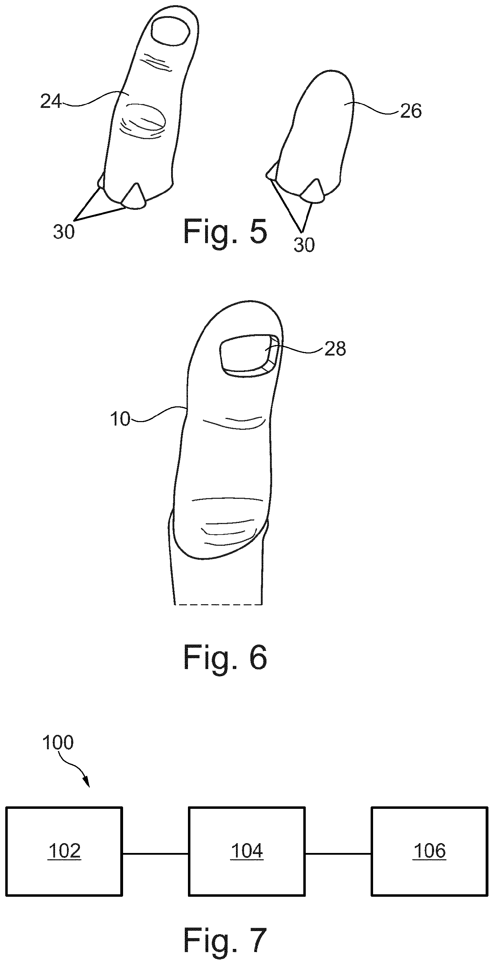

Da die digitalen Abbilder der beiden Finger in feiner Auflösung vorliegen, kann das digitale Abbild der Prothese 22 zudem einfach unter Verwendung eines 3D-Bearbeitungsprogramms am Computer gestaltet werden. Hierzu wird das gespiegelte digitale Abbild des unversehrten Fingers über das digitale Abbild des Fingerstumpfes 20 gelegt und der sich überschneidende Teil aus dem gespiegelten digitalen Abbild des unversehrten Fingers entfernt. Der dann übrigbleibende Teil des digitalen Abbildes des unversehrten Fingers bildet nun die Grundlage für das digitale Abbild der Prothese 22 für den Fingerstumpf. Um einen besseren Sitz zu gewährleisten wird das digitale Abbild des Fingerstumpfes 20 leicht herunterskaliert. Ferner wird nach Bedarf ein ausgesparter Bereich 28 in den Finger eingefügt (siehe Figur 6), indem das Nagelbett des Fingers in dem digitalen Abbild der Prothese 22 freigelegt wird, um in der späteren Prothese 10 einen Kunstnagel aus Acryl darauf zu kleben. Durch den Kunstnagel wird die Prothese noch lebensechter gestaltet.

In einem fünften Verfahrensschritt 58 wird eine Positiv-Form der Prothese 24 und eine Positiv-Form des beeinträchtigten Körperteils 26 unter Verwendung der digitalen Abbilder der Prothese 22 und des beeinträchtigten Körperteils 20 erzeugt (siehe Figur 5). Hierzu wird ein 3D-Drucker mit einer Druckauflösung von kleiner als 0,1 mm, wie einem Kunstharz-3D-Drucker, verwendet. Alternativ kann auch ein SLS-3D-Drucker verwendet werden. Wichtig ist, dass der verwendete Drucker die erforderliche Druckauflösung bereitstellen kann. Insofern kommt es auf letztendlich verwendete 3D-Druckertechnologie nicht an. Erst die Druckqualität des verwendeten Druckers, wie eines SLS- oder Kunstharz-3D-Druckers oder eines 3D-Druckers mit einer vergleichbaren Auflösung, mit dem Schichtdicken zwischen 0,1 bis 0,01 mm erreichbar sind, ermöglicht es, die feinen Oberflächenstrukturen der digitalen Abbilder 20, 22 in die physischen Positiv-Formen zu überführen.

Anhand der ausgedruckten Positiv-Form der Prothese 24 wird in einem sechsten Verfahrensschritt 60 ein Negativ-Abdruck der Prothese erstellt. Der erstellte Negativ-Abdruck der Prothese steht nun zur mehrfachen Verwendung zur Verfügung, um eine Vielzahl von Prothesen 10, beispielsweise auch aus unterschiedlichen Materialen und/oder Farbmischungen, herzustellen. Im Vergleich zu einem direkten Ausdrucken der Prothese 10 mit dem 3D-Drucker ist die Wahl des Werkstoffes und der Farbe nicht auf die durch den 3D-Drucker vorgegebenen Materialien, wie feinkörnige Pulver oder Kunstharze, beschränkt. Folglich können günstigere Werkstoffe, wie Silikonkautschuke, zur Herstellung der Prothese 10 verwendet werden. Silikonkautschuke sind zudem hautverträglich und gut verarbeitbar.

In einem siebten Verfahrensschritt 62 wird mindestens eine Prothese 10 unter Verwendung des Negativ-Abdrucks der Prothese und der Positiv-Form des beeinträchtigten Körperteils 26 hergestellt. Hierzu wird zu vernetzendes Silikon in den Negativ-Abdruck der Prothese gegossen und die Positiv-Form des beeinträchtigten Körperteils 26 in den Negativ-Abdruck der Prothese eingepresst, um den passgenauen Übergang der Prothese 10 zu dem Fingerstumpf auszuformen. Nach der Vernetzung des Silikons bleibt die durch den Negativ-Abdruck vorgegebene Form sowie der durch die Positiv-Form des beeinträchtigten Körperteils 26 vorgegebene Übergang der Prothese 10 erhalte. Da der Negativ-Abdruck mehrfach verwendbar ist, kann durch entsprechend gefärbte Silikone eine Vielzahl von Prothesen 10 für unterschiedliche Hauttöne hergestellt werden. Somit steht dem Patienten eine große Auswahl von verschiedenfarbigen Prothesen offen, die kostengünstig und schnell herstellbar sind.

Auch auf Veränderungen des Fingerstumpfes kann einfach reagiert werden, indem ein neues digitalen Abbild des Fingerstumpfes 20 erstellt und eine neue Positiv-Form des Fingerstumpfes 26 unter Verwendung des 3D-Druckers erstellt wird. In den meisten Fällen muss die Positiv-Form der Prothese 24 nicht verändert, das heißt, neu ausgedruckt werden, sodass der bereits vorhandene Negativ-Abdruck der Prothese erneut verwendet werden kann. Durch das Einpressen der neuen Positiv-Form des Fingerstumpfes 26 in das sich in dem Negativ-Abdruck befindliche Silikon kann eine Prothese 10 mit einem angepassten neuen Übergang zum veränderten Fingerstumpf hergestellt werden.

Wie ferner in Figur 4 dargestellt ist, können Formschlösser 30 in die digitalen Abbilder des beeinträchtigten Körperteils 20 (nicht dargestellt) und der Prothese 22 eingefügt werden. Das Anbringen von Formschlössern 30 der Positiv-Form des beeinträchtigten Körperteils 26 (siehe Figur 5) gewährleistet deren korrekten Sitz in dem Negativ-Abdruck der Prothese, wodurch die Herstellung vereinfacht wird. Bevorzugt sind die Formschlösser 30 derart ausgestaltet, dass nur genau eine Anordnungsmöglichkeit der Positiv-Form des beeinträchtigten Körperteils 26 in dem Negativ-Abdruck der Prothese besteht. Die Formschlösser 30 sind beispielsweise in konischer Form ausgestaltet.

In der vorstehenden Beschreibung ist zu beachten, dass die Verfahrensschritte 50, 52, 58 und 60 grundsätzlich von optionaler Natur für die Erfindung sind. Dieser Umstand wird in Figur 1 dadurch angedeutet, dass die Verfahrensschritte 50, 52, 58 und 60 in einem gestrichelt dargestellten Kästchen abgebildet sind. Beispielsweise können die digitalen Abbilder des Fingerpaares alternativ direkt an dem Fingerpaar des Patienten durch einen 3D-Scanner erzeugt werden. Außerdem ist es möglich, dass die Prothese 10 direkt gemäß dem digitalen Abbild der Prothese 22 ausgedruckt wird.

Figur 7 zeigt schließlich noch eine schematische Darstellung einer Vorrichtung 100 gemäß einer Ausführungsform. Die Vorrichtung 100 ist zum Herstellen einer Prothese 10 für ein Körperteil einer Gliedmaße, insbesondere der im Hinblick auf das Verfahren gemäß Figur 1 und in den Figuren 2 bis 6 beschriebenen Herstellung einer Prothese 10 für einen Fingerstumpf, geeignet.

Die Vorrichtung 100 umfasst eine Erfassungseinheit 102, eine elektronische Steuereinheit 104 und einen 3D-Drucker 106 mit einer Druckauflösung von kleiner als 0,1 mm. Der 3D-Drucker 106 ist bevorzugt ein SLS- oder Kunstharz-3D-Drucker. Die Erfassungseinheit 102 ist zum Erstellen von digitalen Abbildern von beeinträchtigten 20 und unversehrten Körperteilen einer Gliedmaße eingerichtet Die elektronische Steuereinheit 104 ist dazu eingerichtet, ein digitales Abbild einer Prothese 22 für das beeinträchtigte Körperteil unter Verwendung eines gespiegelten digitalen Abbildes des unversehrten Körperteils und eines digitalen Abbildes des beeinträchtigten Körperteils 20 zu erstellen. Der 3D-Drucker 106 ist dazu eingerichtet, mindestens eine Prothese 10 für das beeinträchtigte Körperteil unter Verwendung des digitalen Abbildes der Prothese 22 herzustellen. Dies kann entweder direkt oder indirekt, zum Beispiel unter Hinzuziehung eines Formbaukastens zum Anfertigen von Negativ-Abdrücken (nicht dargestellt), erfolgen.

### Bezugszeichenliste

- 10: Prothese für ein Körperteil einer Gliedmaße
- 12: Negativ-Abdruck eines beeinträchtigten Körperteils
- 14: Negativ-Abdruck des zugehörigen unversehrten Körperteils
- 16: Positiv-Form eines beeinträchtigten Körperteils
- 18: Positiv-Form des zugehörigen unversehrten Körperteils
- 20: Digitales Abbild des beeinträchtigten Körperteils
- 22: Digitales Abbild der Prothese
- 24: Positiv-Form der Prothese
- 26: Positiv-Form des beeinträchtigten Körperteils
- 28: ausgesparter Bereich
- 30: Formschlösser
- 50: erster Verfahrensschritt
- 52: zweiter Verfahrensschritt
- 54: dritter Verfahrensschritt
- 56: vierter Verfahrensschritt
- 58: fünfter Verfahrensschritt
- 60: sechster Verfahrensschritt
- 62: siebter Verfahrensschritt
- 100: Vorrichtung zum Herstellen einer Prothese für ein Körperteil einer Gliedmaße
- 102: Erfassungseinheit
- 104: elektronische Steuereinheit
- 106: 3D-Drucker

## Patentansprüche

1. Verfahren zum Herstellen einer Prothese (10) für ein Körperteil einer Gliedmaße, das die Schritte umfasst:
- Erstellen (54) eines digitalen Abbildes eines beeinträchtigten Körperteils (20) einer Gliedmaße und eines digitalen Abbildes eines zugehörigen unversehrten Körperteils der Gliedmaße,
- Erstellen (56) eines digitalen Abbildes einer Prothese (22) für das beeinträchtigte Körperteil unter Verwendung eines gespiegelten digitalen Abbildes des unversehrten Körperteils und des digitalen Abbildes des beeinträchtigten Körperteils (20),
- Herstellen (62) mindestens einer Prothese (10) für das beeinträchtigte Körperteil unter Verwendung des digitalen Abbildes der Prothese (22) und eines 3D-Druckers (106) mit einer Druckauflösung von kleiner als 0,1 mm.

2. Verfahren nach Anspruch 1, wobei die Prothese (10) für das beeinträchtigte Körperteil direkt aus dem digitalen Abbild der Prothese (22) durch den 3D-Drucker (106) ausgedruckt wird.

3. Verfahren nach Anspruch 1, das ferner die Schritte umfasst:
- Erzeugen (58) einer Positiv-Form der Prothese (24) und einer Positiv-Form des beeinträchtigten Körperteils (26) unter Verwendung der digitalen Abbilder der Prothese (10) und des beeinträchtigten Körperteils und des 3D-Druckers (106),
- Erzeugen (60) eines Negativ-Abdrucks der Prothese unter Verwendung der Positiv-Form der Prothese (24) und
- Herstellen der Prothese (10) für das beeinträchtigte Körperteil unter Verwendung des Negativ-Abdrucks der Prothese.

4. Verfahren nach Anspruch 3, wobei beim Herstellen der Prothese (10) für das beeinträchtigte Körperteil ein Textilstoff in die hergestellte Prothese (10) eingebettet wird.

5. Verfahren nach Anspruch 3 oder 4, wobei Luftlöcher und/oder Formschlösser (30) in die digitalen Abbilder des beeinträchtigten Körperteils (20) und der Prothese (22) eingefügt werden.

6. Verfahren nach einem der vorangehenden Ansprüche, wobei die Prothese (10) für das beeinträchtigte Körperteil ein Entlastungskissen aus einem weichen Silikon umfasst.

7. Verfahren nach Anspruch 6, soweit auf einen der Ansprüche 3 bis 5 rückbezogen, wobei das digitale Abbild des beeinträchtigten Körperteils (20) um eine vorgesehene Dicke des Entlastungskissens verlängert wird und beim Herstellen der Prothese (10) für das beeinträchtigte Körperteil unter Verwendung des Negativ-Abdrucks der Prothese ein erstes Silikon in den Negativ-Abdruck eingegossen, die Positiv-Form des verlängerten beeinträchtigten Körperteils (26) in den Negativ-Abdruck eingepresst wird und nach Aushärtung des ersten Silikons ein zweites Silikon auf das erste Silikon in dem Negativ-Abdruck gegossen wird, wobei das zweite Silikon weicher als das erste Silikon ist.

8. Verfahren nach einem der vorangehenden Ansprüche, wobei eine Farbtonbestimmung der Gliedmaße erfolgt und die Prothese (10) in einer Farbgebung basierend auf dem bestimmten Farbton hergestellt wird.

9. Verfahren nach einem der vorangehenden Ansprüche, wobei der verwendete 3D-Drucker (106) ein SLS- oder Kunstharz-3D-Drucker ist.

10. Verfahren nach einem der vorangehenden Ansprüche, wobei eine Größe des digitalen Abbildes der Prothese (22) und/oder des digitalen Abbildes des beeinträchtigten Körperteils (20) derart angepasst wird, dass die hergestellte Prothese (10) mittels Saugeffekt an dem beeinträchtigten Körperteil befestigbar ist.

11. Verfahren nach einem der vorangehenden Ansprüche, das ferner die Schritte umfasst:
- Erzeugen (50) eines Negativ-Abdrucks des beeinträchtigten Körperteils (12) und eines Negativ-Abdrucks des zugehörigen unversehrten Körperteils (14) und
- Erzeugen (52) einer Positiv-Form des beeinträchtigten Körperteils (16) und einer Positiv-Form des unversehrten Körperteils (18) unter Verwendung der Negativ-Abdrücke (12, 14), wobei die digitalen Abbilder des beeinträchtigten Körperteils (20) und des unversehrten Körperteils unter Verwendung der Positiv-Formen (16, 18) erstellt werden.

12. Verfahren nach einem der vorangehenden Ansprüche, wobei die hergestellte Prothese (10) einen Kern aus einem Silikongemisch mit Füllstoffen umfasst.

13. Verfahren nach einem der vorangehenden Ansprüche, wobei in dem digitalen Abbild der Prothese (22) ein ausgesparter Bereich (28) für ein einzusetzendes Objekt vorgesehen ist.

14. Vorrichtung (100) zum Herstellen einer Prothese (10) für ein Körperteil einer Gliedmaße, umfassend:
- eine Erfassungseinheit (102), die zum Erstellen von digitalen Abbildern von beeinträchtigten (20) und unversehrten Körperteilen einer Gliedmaße eingerichtet ist,
- eine elektronische Steuereinheit (104), die dazu eingerichtet ist, ein digitales Abbild einer Prothese (22) für das beeinträchtigte Körperteil unter Verwendung eines gespiegelten digitalen Abbildes des unversehrten Körperteils und eines digitalen Abbildes des beeinträchtigten Körperteils (20) zu erstellen, und
- einen 3D-Drucker (106) mit einer Druckauflösung von kleiner als 0,1 mm, der dazu eingerichtet ist, mindestens eine Prothese (10) für das beeinträchtigte Körperteil unter Verwendung des digitalen Abbildes der Prothese (22) herzustellen.

15. Vorrichtung (100) nach Anspruch 14, ferner umfassend einen Formbaukasten zum Anfertigen von Negativ-Abdrücken.
